# EUROPEAN PATENT APPLICATION

(11) **EP 4 793 342 A2**
(43) Date of publication of application: **19.08.2026**
(21) Application number: 26152969.7
(22) Date of filing: 20.01.2026
(51) Int. Cl.: C12M 1/00, C12M 1/12, C12M 1/26, C12M 1/34, C12M 1/36, G01N 35/00, G01N 35/02, G16H 10/40

(54) **CELL HANDLING APPARATUS**

(30) Priority: 28.01.2025 JP 2025012180
(71) Applicant: Yamaha Hatsudoki Kabushiki Kaisha, Iwata-shi, Shizuoka 438-8501 (JP)
(72) Inventor: SHIBUYA, Satoshi, Shizuoka, 4388501 (JP); WATANABE, Masahiko, Shizuoka, 4388501 (JP)
(74) Representative: Müller-Boré & Partner Patentanwälte PartG mbB

(57) **Abstract**

A cell handling apparatus includes: a first plate including a first cell containing portion capable of containing a cell; a second plate including a second cell containing portion capable of containing a cell; a head that sucks a cell from the first cell containing portion, discharges a sucked cell to the second cell containing portion, and transfers a cell between the first plate and the second plate; and a recognition processing unit that recognizes a cell in the first cell containing portion and the second cell containing portion. The recognition processing unit performs processing of assigning a first identification code for recognizing a first cell contained in the first cell containing portion, a second identification code for recognizing the first cell transferred to the second cell containing portion, and cooperation information for associating the first identification code and the second identification code.

## Description

### FIELD OF INVENTION

The present invention relates to a cell handling apparatus having a function of transferring a cell between a plurality of plates.

### BACKGROUND ART

For example, in the fields of medical and biological research, an operation of picking a cell contained in a well of one plate and transferring the cell to a well of another plate may be performed. In this case, it is desirable to assign some management information for identification of a cell to be transferred between plates. Japanese Patent Application Laid-Open No. 2023-108459 discloses a cell handling apparatus that sets an identification code (ID) to each of a plurality of wells included in a plate and updates a well position ID as needed when a cell is transferred between the wells.

In management based on the well position ID assigned in units of wells of a plate as in the above-described apparatus, there has been a case where, when a cell is transferred between plates, management tracking a history of a cell is difficult to perform. If an ID is assigned to a cell itself to be transferred, management may be performed even in a case where transfer between plates is performed. However, cells may divide and increase in number during culture. For example, one cell picked from one plate may divide after transfer to another plate, and the number of cells may increase. In this case, there has been a problem that cell counts exhibit a one-to-many relationship between a transfer source and a transfer destination, and management of cells becomes difficult.

### SUMMARY OF THE INVENTION

An object of the present invention is to accurately perform management of a cell in a cell handling apparatus that transfers a cell between a plurality of plates.

A cell handling apparatus according to one aspect of the present invention includes: a first plate including a first cell containing portion capable of containing a cell; a second plate including a second cell containing portion capable of containing a cell; a head that sucks a cell from the first cell containing portion, discharges a sucked cell to the second cell containing portion, and transfers a cell between the first plate and the second plate; and a recognition processing unit configured to recognize a cell in the first cell containing portion and the second cell containing portion, in which the recognition processing unit performs processing of assigning a first identification code for recognizing a first cell contained in the first cell containing portion, a second identification code for recognizing the first cell transferred to the second cell containing portion, and cooperation information for associating the first identification code and the second identification code.

### BRIEF DESCRIPTION OF THE DRAWINGS

Fig. 1 is a diagram schematically illustrating a configuration of a cell handling apparatus according to an embodiment of the present invention;
Fig. 2A is a perspective view of a plate having a well as a cell containing portion, Fig. 2B is a plan view of one well, and Fig. 2C is a cross-sectional view of a grid;
Fig. 3 is a block diagram illustrating an electrical configuration of the cell handling apparatus;
Figs. 4A and 4B are perspective views illustrating examples of transfer patterns of a cell between plates;
Fig. 5 is a diagram schematically illustrating a conventional management mode of a cell transferred between plates;
Fig. 6 is a diagram schematically illustrating a management mode in this embodiment for a cell transferred between plates;
Fig. 7 is a diagram illustrating, in a form of a tree diagram, patterns A, B, and C for displaying an image of a cell group associated with a cell as a focus target;
Fig. 8 is a diagram illustrating a configuration of a tree diagram of the pattern A illustrated in Fig. 7;
Fig. 9 is a diagram illustrating a configuration of a tree diagram of the pattern B illustrated in Fig. 7; and
Fig. 10 is a diagram illustrating a configuration of a tree diagram of the pattern C illustrated in Fig. 7.

### DETAILED DESCRIPTION

Hereinafter, an embodiment of the present invention will be described in detail with reference to the drawings. A cell handling apparatus according to the present invention is an apparatus that sucks various biologically derived cells from a first plate, transfers the cells to a second plate, and discharges the cells. Examples of the biologically derived cells include single cells such as a blood cell, a dissociated cell, and a fertilized egg, a circulating tumor cell, small tissue pieces such as histoculture, cell aggregates such as a spheroid and an organoid, organisms such as a zebrafish and a nematode, and a 2D or 3D cell colony. In the present specification, the term "cell" includes these various cells.

### [Overall configuration of cell handling apparatus]

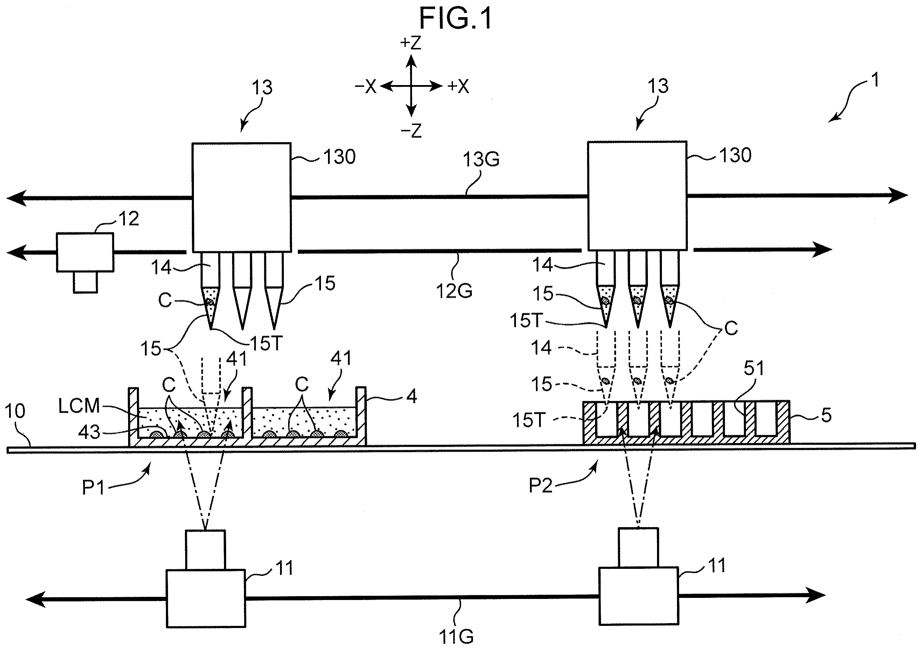

Fig. 1 is a diagram schematically illustrating an overall configuration of a cell handling apparatus 1 according to an embodiment of the present invention. Here, the cell handling apparatus 1 that transfers a cell C between two containers, that is, between a first plate 4 and a second plate 5 is illustrated. As a matter of course, the cell handling apparatus 1 may be configured to transfer the cell C between three or more containers.

The cell handling apparatus includes a translucent base 10 having a horizontal placement surface, a camera 11 disposed below the base 10, and an illumination device 12 and a head unit 13 disposed above the base 10. The first plate 4, which is a transfer source of the cell C, is placed at a first placement position P1 of the base 10, and the second plate 5, which is a transfer destination of the cell C, is placed at a second placement position P2. As a matter of course, the second plate 5 may be a transfer source of the cell C, and the first plate 4 may be a transfer destination of the cell C.

The camera 11 captures an image of the first plate 4 or the second plate 5 containing the cell C. The illumination device 12 irradiates the first plate 4 or the second plate 5 with illumination light at the time of image capturing by the camera 11. The head unit 13 has a function of transferring the cell C between the first plate 4 and the second plate 5. The head unit 13 includes a plurality of heads 14 movable in a Z direction which is a vertical direction. Those heads 14 can be operated simultaneously or individually. A tip 15 for sucking and discharging the cell C is attached to a lower end of each of the heads 14. The camera 11, the illumination device 12, and the head unit 13 are movable in an X direction which is a left-right direction and a Y direction perpendicular to the plane of the drawing of Fig. 1.

Generally, the cell handling apparatus 1 executes the following cell transfer sequence by an operation of the head unit 13 having the head 14 on which the tip 15 is mounted. First, a picking operation of individually sucking a specific target cell C with each of a plurality of the tips 15 from the first plate 4 (well 41) culturing a large number of the cells C is performed. As the head unit 13 moves, the picked cell C is transferred from the first plate 4 to the second plate 5. The cell C sucked into the tip 15 is discharged to the second plate 5 (well 41). Before picking of the cell C, the camera 11 images the cell C held on the first plate 4 irradiated with illumination light of the illumination device 12. Based on an image acquired by the imaging, a high-quality target cell C to be transferred to the second plate 5 is selected.

Hereinafter, each part of the cell handling apparatus 1 will be described. The base 10 is a flat plate that has predetermined rigidity and is partially or entirely made from a translucent material. The preferred base 10 is a glass plate. As the base 10 is formed from a translucent material such as a glass plate, the camera 11 disposed below the base 10 can image the first plate 4 or the second plate 5 disposed on an upper surface of the base 10 through the base 10.

The first plate 4 has a plurality of the wells 41 (first cell containing portions) capable of containing the cell C. Each of the wells 41 is a small container having an upper surface opening and has a flat bottom surface 43. Into the well 41, a liquid medium LCM is injected, and a test cell is seeded. On the bottom surface 43, the seeded cell C is disposed in an adherent or suspension state. For the first plate 4, a member made from a translucent resin material or glass is used in order to enable imaging of the cell C by the camera 11 disposed below. As the first plate 4, for example, a commercially available 6-well plate (for example, model number 4444 manufactured by Corning Incorporated) can be used.

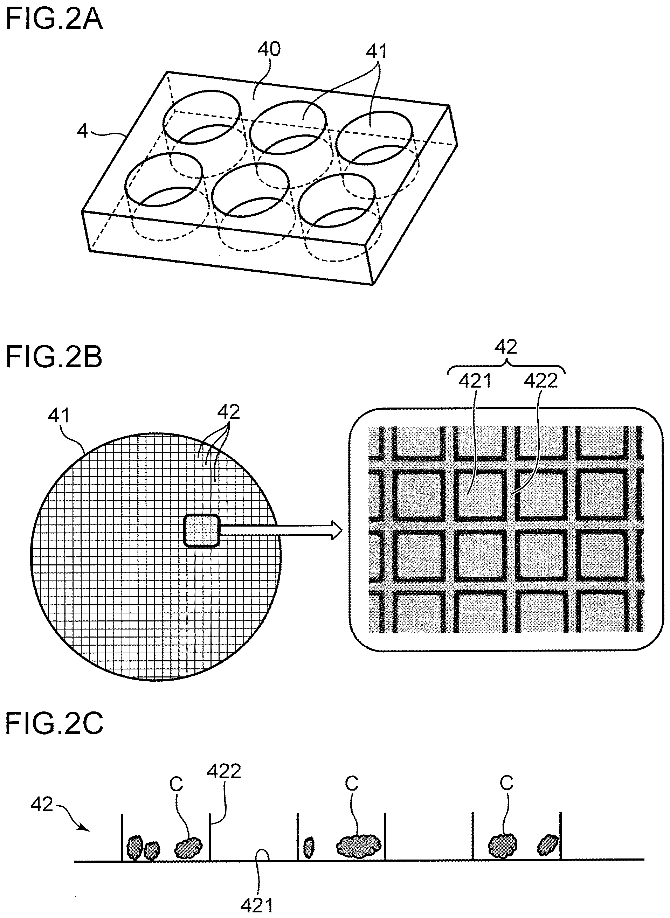

Fig. 2A is a perspective view illustrating an example of the first plate 4 including the above-described 6-well plate. The first plate 4 includes a plate-like plate body 40 and a plurality of the wells 41. The well 41 is a cylindrical bottomed hole formed in the plate body 40. The cell C is dispensed to each of the wells 41 from a dispenser or the like. Fig. 2A illustrates the first plate 4 in which the wells 41 arranged in two rows and three columns are provided in the plate body 40.

Fig. 2B is a plan view of one of the wells 41. A large number of grids 42 are arranged on a bottom surface of the well 41. The grid 42 is formed by a square bottom surface 421 and a partition wall 422 surrounding a periphery of the bottom surface 421. One of the grids 42 is formed of a rectangular parallelepiped having the bottom surface 421 with one side of 200 µm and a height of about 100 µm. In Fig. 2B, the grids 42 arranged in a lattice shape are illustrated. The grid 42 may have another shape, for example, a rectangular shape or a honeycomb shape.

Fig. 2C is a longitudinal cross-sectional view of the grid 42. The bottom surface 421 is a substantially horizontal surface. The partition wall 422 is erected vertically upward from the bottom surface 421. One of the grids 42 is a containing unit of the cell C. One or a plurality of the cells C are contained in one of the grids 42. The grid 42 not containing the cell C also occurs. Each of the grids 42 is treated as a picking unit of the cell C by the tip 15.

The second plate 5 has a plurality of wells 51 (second cell containing portions) capable of containing the cells C. The well 51 is a bottomed hole opened in an upper surface of the second plate 5. The cell C sucked from the wells 41 of the first plate 4 is discharged to the well 51. One of the wells 51 contains the necessary number of the cells C together with a liquid medium. Various tests such as addition of a reagent or a reactant, observation, culture, and the like are performed on the cell C contained in the well 51. A member made from a translucent resin material or glass is also used as the second plate 5. As the second plate 5, for example, a commercially available 96-well plate (for example, model number 3595 manufactured by Corning Incorporated) can be used.

The camera 11 images the cell C held at a bottom portion of the first plate 4 or the second plate 5 from the lower surface side of the plate. The camera 11 includes an objective lens for an optical microscope and an imaging element such as a CMOS sensor. The camera 11 is movable in the X direction below the base 10 along a guide rail 11G extending in the left-right direction in parallel with the base 10. The camera 11 is also movable in the Y direction orthogonal to the X direction on a horizontal plane. The camera 11 of the present embodiment can execute normal bright-field imaging and fluorescence imaging for imaging the fluorescently labeled target cell C.

The illumination device 12 irradiates the first plate 4 or the second plate 5 with illumination light when the camera 11 images the cell C. The illumination device 12 can irradiate the first plate 4 or the second plate 5 with a plurality of types of illumination light. Examples of a plurality of types of illumination light include visible light illumination light for bright-field imaging of the cell C, and short-wavelength illumination light such as an ultraviolet ray and an X-ray for fluorescence imaging of the cell C. The illumination device 12 is movable in the XY directions along a guide rail 12G above the base 10.

The head unit 13 includes a plurality of the heads 14 and a unit body 130 to which the heads 14 are assembled. The unit body 130 holds the head 14 movably in +Z and -Z directions (vertical direction). The unit body 130 is movable in +X and -X directions (horizontal direction) along a guide rail 13G above the base 10. Note that the unit body 130 is also movable in the Y direction. That is, the head 14 is movable in three-dimensional directions of X, Y, and Z.

The tip 15 is attached to a tip of each of the heads 14. The tip 15 has a tip opening 15T that sucks and discharges the cell C. At the time of picking of the cell C, the head 14 is moved so that the tip opening 15T of the tip 15 faces the target cell C from above. After the above, by generating negative pressure in the tip opening 15T, the target cell C is sucked into the tip 15. At the time of discharge of the sucked cell C, positive pressure is applied to the tip opening 15T.

### [Electrical configuration of cell handling apparatus]

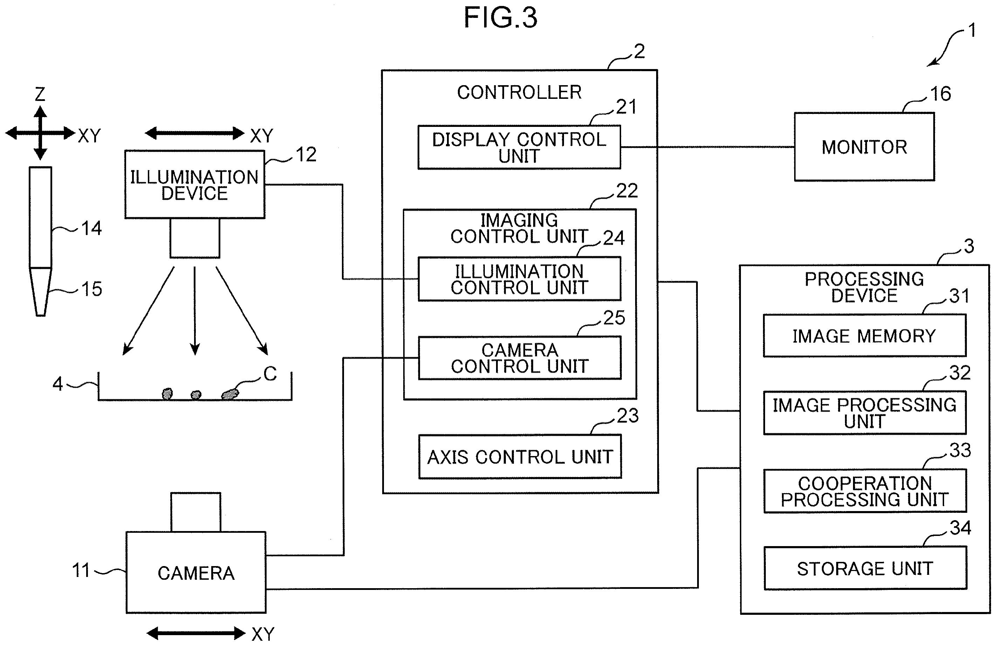

Fig. 3 is a block diagram illustrating an electrical configuration of the cell handling apparatus 1. The cell handling apparatus 1 includes a monitor 16 (display unit), a controller 2, and a processing device 3 (recognition processing unit) in addition to the camera 11, the illumination device 12, and the head 14 described above.

The monitor 16 displays various types of information necessary for an operation of the cell handling apparatus 1. In the present embodiment, information on the cell C to be handled and information on various cells necessary for recognizing the cell C to be a sucking target are displayed on the monitor 16. The information includes cooperation information that can track a history of the cell C in a case where the cell C is transferred between plates. The cooperation information is association information for realizing, for example, lineage-style display in a case where the number of the cells C increases due to division, linear lineage display in which a certain child cell is focused and reaches a parent cell, lineage-style display indicating a relationship between a plurality of parent cells and an aggregate cell, and the like.

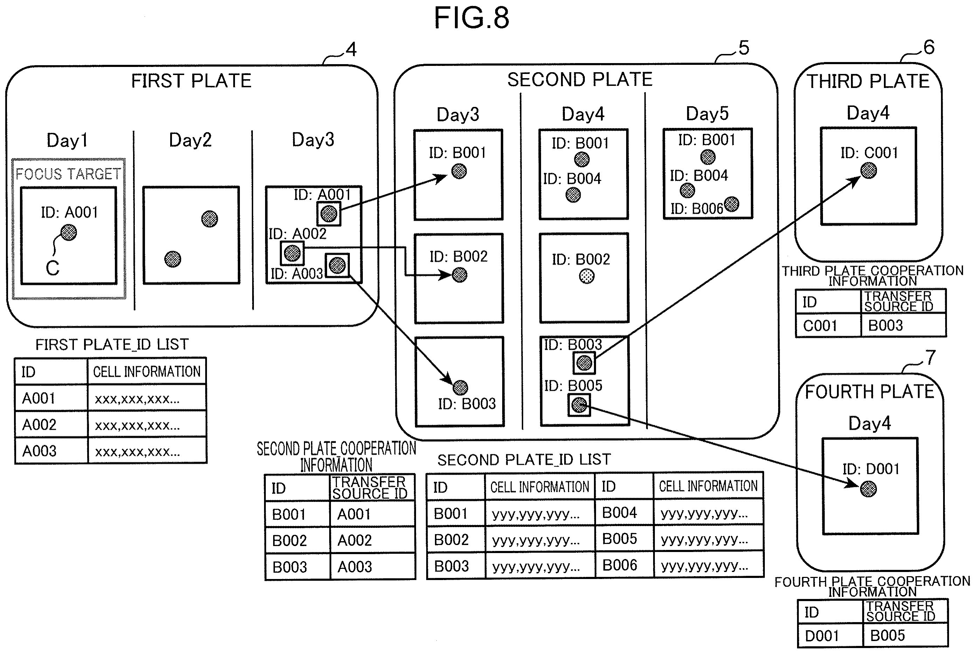

The controller 2 includes a processor and the like, and integrally controls an operation of each unit of the cell handling apparatus 1. The controller 2 operates to functionally include a display control unit 21, an imaging control unit 22, and an axis control unit 23 by reading a predetermined program. The display control unit 21 controls a display operation on the monitor 16. In the present embodiment, the display control unit 21 controls a display operation of various types of cell information based on a processing result of the processing device 3. A specific example of the display operation will be described with reference to Figs. 8 to 10.

The imaging control unit 22 controls an imaging operation of the cell C in the first plate 4 or the second plate 5. The imaging control unit 22 includes an illumination control unit 24 and a camera control unit 25. The illumination control unit 24 controls an operation of the illumination device 12. Specifically, the illumination control unit 24 controls selection of illumination light emitted from the illumination device 12, a lighting timing of illumination light, and the like. The camera control unit 25 controls an operation of the camera 11. Specifically, the camera control unit 25 controls an exposure amount, a shutter timing, a focusing operation, and the like of the camera 11.

The axis control unit 23 controls a movement operation of the camera 11, the illumination device 12, the head unit 13, and the head 14. The axis control unit 23 moves the camera 11 and the illumination device 12 to predetermined positions along the guide rails 11G and 12G by controlling a drive motor for XY axis movement of the camera 11 and the illumination device 12. Further, the axis control unit 23 controls a drive motor for XY axis movement of the head unit 13 and a drive motor for Z axis movement of the head 14 to cause the head 14 equipped with the tip 15 to execute a picking operation of the cell C.

The processing device 3 executes processing of recognizing the cell C, processing of assigning cooperation information, and the like. The processing of recognition is processing of identifying the cell C in the first plate 4 or the second plate 5 based on an image acquired by the camera 11. The cooperation information is a procedure of assigning information for associating a plurality of the recognized cells C with each other. The processing device 3 includes an image memory 31, an image processing unit 32, a cooperation processing unit 33, and a storage unit 34.

The image memory 31 is a memory region that temporarily stores image data captured by the camera 11. The image processing unit 32 performs image processing such as edge detection processing and pattern recognition processing accompanied by feature amount extraction on image data stored in the image memory 31. By this image processing, image recognition of a cell contained in the grid 42 which is a cell containing unit of the first plate 4, image recognition of a cell in the wells 51 of the second plate, and the like are performed.

The cooperation processing unit 33 performs processing of assigning information indicating connection of various cells recognized by image processing of the image processing unit 32. For example, the cooperation processing unit 33 performs processing of assigning a unique identification code to each of recognized cells and processing of assigning cooperation information for associating the identification codes with each other. The identification code and the cooperation information are also assigned to each of cells that divide after transfer between plates. The storage unit 34 stores a database including the identification code, the cooperation information, a feature amount of each cell, and the like, image information of a cell, and the like. The display control unit 21 of the controller 2 appropriately reads data from the storage unit 34 and causes the monitor 16 to display required cell information.

### [Conventional example and example of cell management]

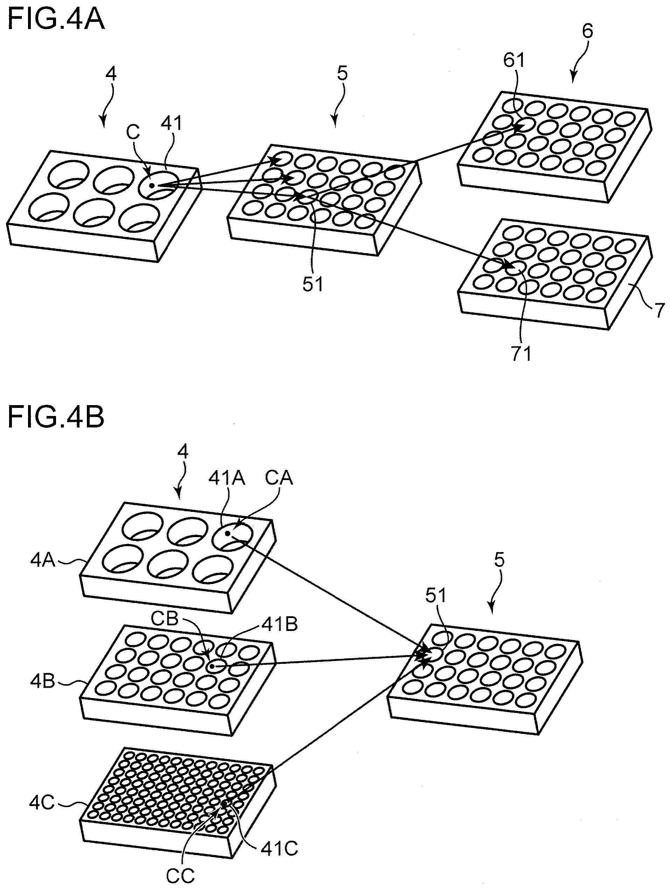

Figs. 4A and 4B are perspective views illustrating an example of a transfer pattern of the cell C between plates. Fig. 4A is an example in which the cell C is transferred between four plates, the first plate 4, the second plate 5, a third plate 6, and a fourth plate 7. After the cells C are cultured in the well 41 of the first plate 4 for a predetermined period, the cells C are transferred to some of the wells 51 of the second plate 5. In the well 51, the cell C is cultured for a predetermined period. At this time, the cell C may divide and increase in number. After the above, the cell C in the well 51 is transferred to a well 61 of third plate 6 and a well 71 of the fourth plate 7.

Fig. 4B illustrates an example in which the cells C are transferred from a plurality of the first plates 4 to one second plate 5. A plurality of the first plates 4 includes three plates 4A, 4B, and 4C. Cells CA, CB, and CC are contained in wells 41A, 41B, and 41C of the plates 4A, 4B, and 4C, respectively. The cells CA, CB, and CC are, for example, element cells forming an artificial organ. The cells CA, CB, and CC are picked from the wells 41A, 41B, and 41C of the plates 4A, 4B, and 4C, respectively, and transferred to one of the wells 51 of the second plate 5.

It is easy to manage cells in units of one cell containing portion of one plate. For example, in a case where the cell C contained in each of the grids 42 of the well 41 of the first plate 4 is managed, an identification code is preferably assigned to the cell C existing in the grid 42, or an identification code is preferably assigned to the grid 42 itself. However, in a case where the cell C is transferred between plates as illustrated in Figs. 4A and 4B, a case where the cell C divides after transfer, or the like, management of cell information may be difficult.

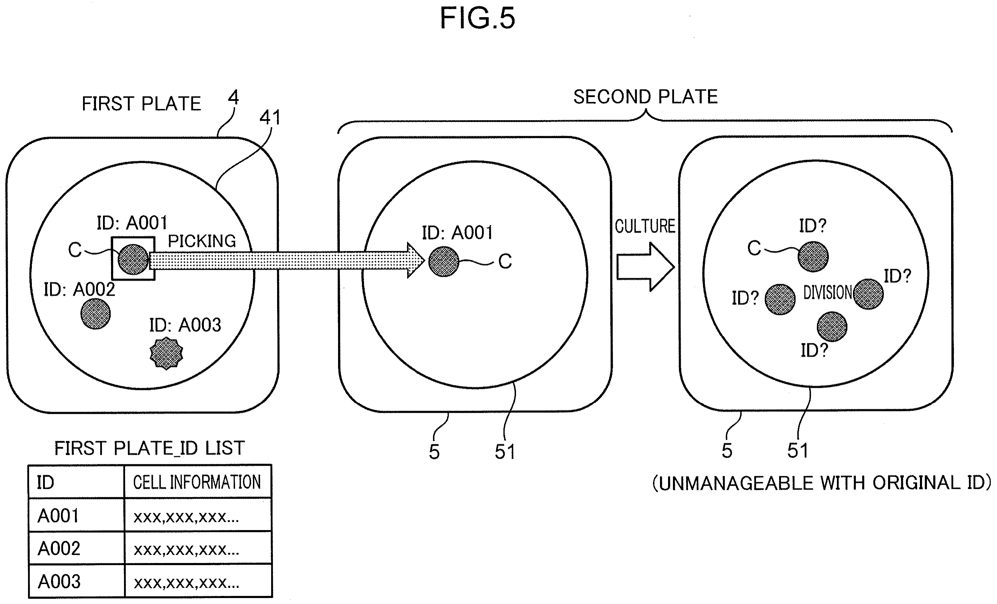

Fig. 5 is a diagram schematically illustrating a conventional management mode of a cell transferred between plates. In the diagram, the first plate 4 and one of the wells 41, and the second plate 5 and one of the wells 51 are schematically illustrated. Three of the cells C contained in the well 41 of the first plate 4 are assigned with IDs =A001, A002, and A003 as identification codes. In an ID list of the first plate 4, cell information of each of the cells C is managed in association with each ID of A001, A002, and A003. The cell information is, for example, cell feature amounts, such as contour data, an in-well position, fluorescence intensity, concentration, and circularity, and size information such as an area, a diameter, and an aspect ratio of a cell, which are extracted based on an image of the cell C.

In the example of Fig. 5, the cell C with ID = A001 is picked from the well 41 and transferred to the well 51 of the second plate 5. At a time point of this transfer, the cell C after the transfer can be managed by using an identification code of ID = A001, address data of the well 51 to which the cell C is discharged, or the like. However, during culture on the second plate 5, the cell C of A001 may divide and increase in number. In this case, management with a single ID of "A001" is no longer possible.

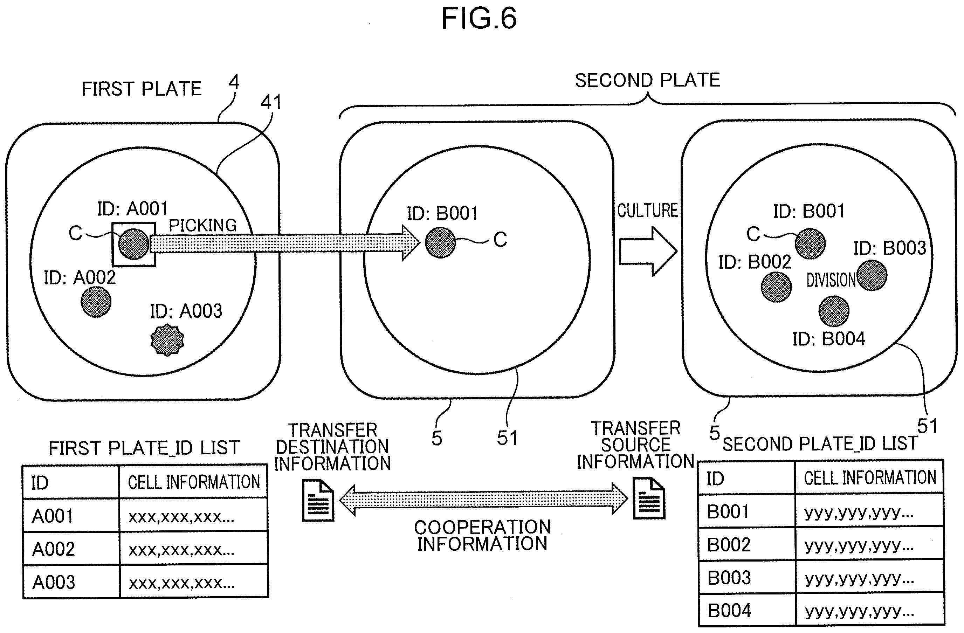

Fig. 6 is a diagram schematically illustrating a management mode in this embodiment for a cell transferred between plates. Three of the cells C (first cells) contained in the well 41 (first cell containing portion) of first plate 4 are assigned with ID = A001, A002, and A003 (first identification codes) as identification codes. In association with these IDs, cell information of each of the cells C is managed. The above points are the same as in a conventional technique.

Subsequently, it is assumed that the cell C with ID = A001 is picked from the well 41 and transferred to the well 51 of the second plate 5. In this case, the cooperation processing unit 33 (Fig. 3) assigns ID = B001 (second identification code) as a new identification code to the transferred cell C. Furthermore, the cooperation processing unit 33 provides cooperation information for associating ID = A001 and ID = B001. Further, in a case where the cell C divides in the well 51 after culture, that is, in a case where the first cell divides into at least a second cell and a third cell, different identification codes are newly assigned to the divided cells C. In the example of Fig. 6, four of the divided cells C are assigned with different IDs = B001, B002, B003, and B004 as identification codes. In this case, the cooperation processing unit 33 assigns cooperation information for associating ID = A001 and IDs = B001 to B004. In an ID list of the second plate 5, cell information of each of the cells C of IDs = B001 to B004 is managed.

Even in a case where an identification code of the cell C changes before and after transfer between plates, a relationship between both cells can be tracked by assigning processing of cooperation information. The cooperation information can be created, for example, by describing a relationship between identification codes of both cells in an ID list of the first plate 4 and an ID list of the second plate 5. Specifically, in an ID list of the first plate 4, it is described that an identification code at a transfer destination of the cell C with ID = A001 is ID = B001 or ID = B001 to B004. Further, in an ID list of the second plate 5, it is described that an identification code at a transfer source of the cell C with ID = B001 or ID = B001 to B004 is ID = A001. By assigning such cooperation information, even in a case where the cell C is transferred between plates, the lineage can be traced, so that the cell C can be accurately managed.

A timing at which an identification code is assigned to the cell C transferred to the well 51 of the second plate 5 may be a timing at which the well 51 is imaged and image processing is performed on the acquired image data to identify the cell C. The well 51 is imaged, for example, at a timing of performing some action that requires management, such as picking, observing, or administering a reagent or the like to the cell C in the well 51. That is, when the cell C in the well 51 is not divided at the timing of performing the action, ID = B001 is assigned to the one cell C as an identification code. On the other hand, when the cell C divides into four at the timing of the action, IDs = B001, B002, B003, and B004 are assigned to the divided cells C as identification codes.

### [Display example of cell information]

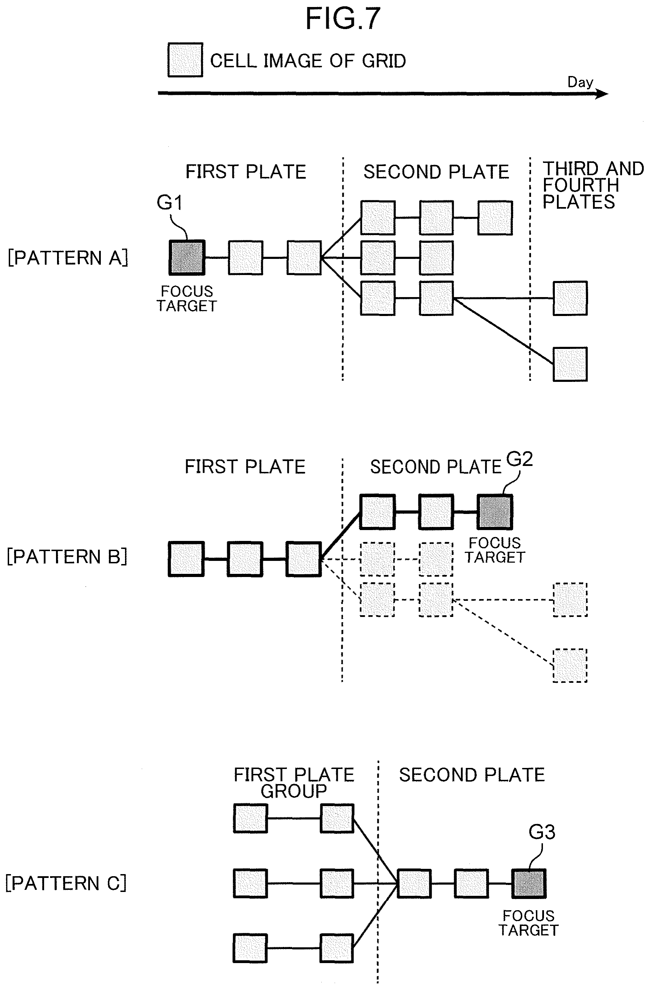

Fig. 7 illustrates a display example in a case where the display control unit 21 of the controller 2 displays an image of a cell group associated with a cell as a focus target on the monitor 16 in a form of a tree diagram. Fig. 7 illustrates Patterns A, B, and C as typical display examples. A square block in the diagram shows an image (hereinafter, referred to as a grid image) of one of the grids 42 (Fig. 2) containing the cell C. The horizontal axis toward the left indicates passage of date and time of culture of the cell C.

Pattern A is an example of a lineage-style tree diagram representing an entire state of subsequent cell division starting from a grid image G1 obtained by imaging an initial state of the cell C as a division source. Pattern A assumes that the cell C is sequentially transferred from the first plate 4 to the second plate 5 and from the second plate 5 to the third plate 6 and the fourth plate 7 as culture proceeds as illustrated in Fig. 4A.

Pattern B is an example of a tree diagram linearly showing a lineage that has a grid image G2 obtained by imaging a state of the terminal cell C after division as a starting point, and reaches its own parent cell. Pattern B also assumes culture and a transfer example of the cell C illustrated in Fig. 4A.

Pattern C is an example of a lineage-style tree diagram in which a grid image G3 obtained by imaging an aggregate cell formed by a plurality of cells is set as a starting point, and an entire relationship between the aggregate cell and a plurality of parent cells is shown. As illustrated in Fig. 4B, Pattern C assumes that cells CA, CB, and CC picked from three of the first plates 4A, 4B, and 4C are transferred to the one second plate 5. Hereinafter, a specific display example of the tree diagrams of Patterns A, B, and C on the monitor 16 will be described.

### <Pattern A>

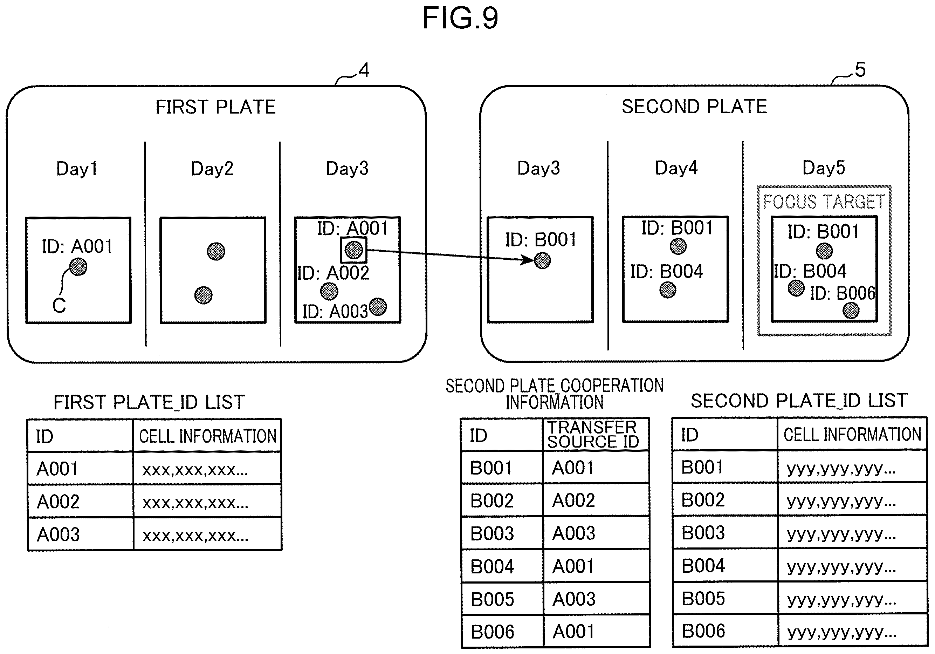

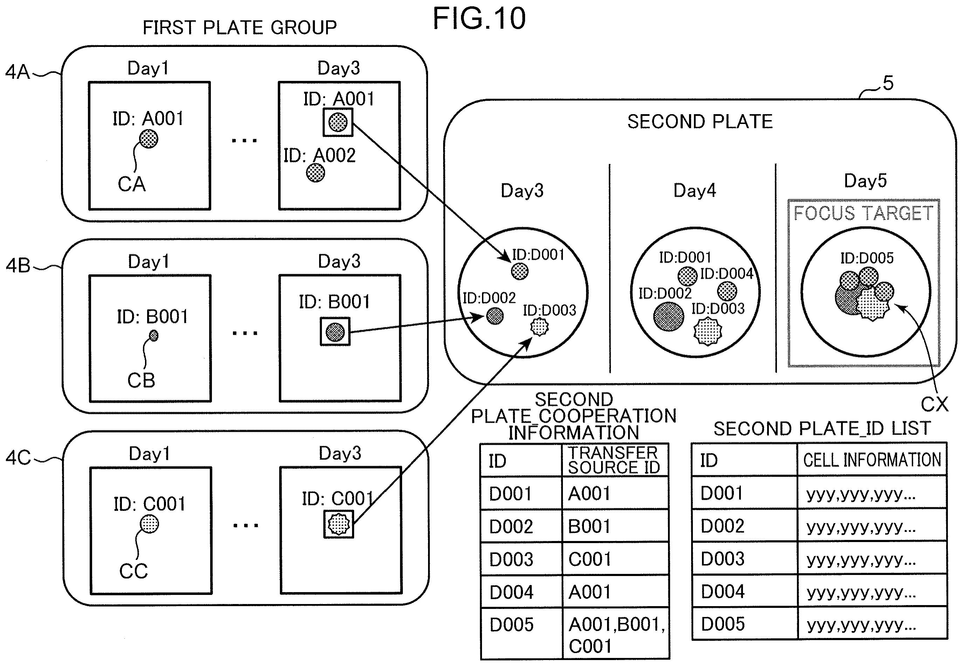

Fig. 8 is a diagram illustrating a specific example of the tree diagram of Pattern A illustrated in Fig. 7. In Fig. 8 and subsequent Figs. 9 and 10, a square block in the drawing shows one grid image containing the cell C as in Fig. 7. Further, display of "Day 1", "Day 2", ... represents the number of days of culture of a cell.

One cell C is observed in a grid image on the first day of culture on Day 1 of the first plate 4. The cooperation processing unit 33 (Fig. 3) of the processing device 3 assigns ID = A001 as an identification code to the one cell C. In the pattern A, the grid image on Day 1 is a focus target. Fig. 8 shows a grid image in which the cell C is divided into two on Day 2 by culture in the first plate 4. The cooperation processing unit 33 assigns IDs = A001 and A002 as identification codes to two of the cells C. On Day 3, since the cells divide into three of the cells C, the cooperation processing unit 33 assigns IDs = A001, A002, and A003 as identification codes to the three of the cells C.

As described above, in actual identification code assignment processing, an identification code is assigned to the cell C existing in the grid 42 at an action timing such as picking with respect to the grid 42 as a focus target. Fig. 8 illustrates an example in which Day 3 is set as a picking execution date for the cell C in the grid 42 as a focus target. In this case, processing of assigning an identification code on Day 2 is not executed. On the other hand, on Day 3, processing of assigning an identification code to the cell C existing in the grid 42 is executed before picking. Further, the cooperation processing unit 33 creates an ID list for the grid 42 as a focus target of the first plate 4. As illustrated in Fig. 8, the ID list is a list in which the cells C to which IDs = A001, A002, and A003 are assigned and cell information extracted based on a grid image of Day 3 are described. The ID list is also cell division information indicating that one of the cells C existing in the grid 42 as a focus target on Day 1 divides into three of the cells C with IDs = A001, A002, and A003.

On Day 3, the cell C existing in the grid 42 as a focus target is transferred from the first plate 4 to the second plate 5. The cells C with ID= A001, A002, and A003 are individually transferred to three of the grids 42 of the second plate 5. A grid image on Day 3 of the second plate is a grid image after transfer of the cells C with IDs = A001, A002, and A003.

The cooperation processing unit 33 newly assigns IDs = B001, B002, and B003 as identification codes to these three of the cells C. Further, the cooperation processing unit 33 creates, as an ID list of the second plate, a list in which the cells C to which the IDs = B001, B002, and B003 are assigned and cell information of the cells are described. Furthermore, the cooperation processing unit 33 creates a list of cooperation information that associates the IDs = A001, A002, and A003 as the first identification codes with the IDs = B001, B002, and B003 as the second identification codes, respectively. The cooperation information list is inter-plate transfer information indicating a transfer path of the cell C from the first plate 4 to the second plate 5.

On Day 4 in culture on the second plate 5, the cells C with ID = B001 and ID = B003 divide into two. That is, three of the cells C with IDs = B001, B002, and B003 transferred to the second plate on Day 3 increase to a total of five of the cells C on Day 4. In a case where Day 4 is the action timing, IDs = B001 to B005 are assigned as identification codes to these five of the cells C. Furthermore, on Day 5, the cell C with ID = B001 on Day 3 divides into three. In a case where Day 5 is the action timing, the cooperation processing unit 33 creates a list in which the cells C to which IDs = B001 to B006 are assigned and cell information of the cells are described. This list is also cell division information.

On Day 4, the cell C with ID = B003 is transferred to the third plate 6, and the cell C with ID = B005 is transferred to the fourth plate 7. The cooperation processing unit 33 newly assigns ID = C001 to the cell C transferred to the third plate 6 and newly assigns ID = D001 to the cell C transferred to the fourth plate 7 as identification codes. Further, the cooperation processing unit 33 creates, for the third plate 6, a list of cooperation information that associates ID = B003 of a transfer source with ID = C001. For the fourth plate 7, a list of cooperation information that associates ID = B005 of a transfer source with ID = D001 is created. These cooperation information lists are inter-plate transfer information indicating transfer paths of the cells C from the second plate 5 to the third plate 6 and the fourth plate 7. Note that, although not illustrated, an ID list describing cell information on cells with ID = C001 and ID = D001 is also created.

The tree diagram illustrated in Fig. 8 is a tree diagram that the display control unit 21 causes the monitor 16 to display based on information regarding a cell provided by the cooperation processing unit 33 as described above. The tree diagram represents, in a form of lineage, how the cell C as a focus target is transferred between plates after picking, and divides and differentiates. For this reason, the user who visually recognizes the monitor 16 can grasp at a glance a differentiation process of the cell C sequentially transferred from the first plate 4 to the second plate 5, and further to the third plate 6 and the fourth plate 7.

### <Pattern B>

Fig. 9 is a diagram illustrating a specific example of a tree diagram of Pattern B illustrated in Fig. 7. In an inter-plate transfer situation and a differentiation process of the cells C identical to those in Pattern A, Pattern B sets a grid image on Day 5 in the second plate 5 as a focus target. It is assumed that an ID list of the first plate 4, an ID list of the second plate 5, and a cooperation information list which are created by the cooperation processing unit 33 are also identical to those in Pattern A.

When a grid image on Day 5 of the second plate 5 is set as a focus target and a command to display the origin of the cell C (designated cell) included in the grid image is given, the display control unit 21 causes the monitor 16 to display the tree diagram illustrated in Fig. 9. The tree diagram of Fig. 9 is a linear tree diagram illustrating that the cells C with IDs = B001, B004, and B006 of the second plate are transferred from the first plate 4 and originated from ID = A001. That is, the tree diagram illustrates inter-plate transfer information of the cell C and linear transition from the cell C with ID = A001 to the cell C with ID = B001, B004, or B006.

Such a tree diagram can be displayed on the monitor 16 since an ID list is created by assignment of an identification code to each of the cells C before and after division, and a cooperation information list that associates IDs = A001 to A003 of the first plate 4 and IDs = B001 to B006 of the second plate is created. The user who visually recognizes the monitor 16 can grasp a lineage after several generations after picking and division are repeated for the cell C as a focus target. Therefore, a tree diagram of Fig. 9 can be utilized to prove an important genetic origin, for example, in establishment of a cell line.

### <Pattern C>

Fig. 10 is a diagram illustrating a specific example of a tree diagram of Pattern C illustrated in Fig. 7. Pattern C assumes a case where cells picked from a plurality of the first plates 4 are transferred to one of the wells 51 of the second plate 5, and one aggregate cell is generated. On Day 1 of a first plate group, the cells CA, CB, and CC are recognized in grid images of the first plates 4A, 4B, and 4C, respectively. On Day 3 of the first plate group, the cell CA with ID = A001 divides into two. In a case where Day 3 is an action timing, the cooperation processing unit 33 assigns IDs = A001 and A002 to two of the cells CA in a grid image of the first plate 4A, and assigns IDs = B001 and C001 to the cells CB and CC in grid images of the other first plates 4B and 4C, respectively.

On Day 3, the cells CA, CB, and CC with IDs = A001, B001, and C001 are picked from the first plate group and transferred to the second plate 5. The cooperation processing unit 33 newly assigns IDs = D001, D002, and D003 to the cells CA, CB, and CC transferred to the second plate 5, respectively. On Day 4, the cell CA divides into two, and the identification codes of ID = D001 and D004 are assigned to the cells CA. Furthermore, on Day 5, the cells CA, CB, and CC form a cell aggregate, so that one aggregate cell CX is generated.

The cooperation processing unit 33 newly assigns ID = D005 as an identification code to the aggregate cell CX. Further, the cooperation processing unit 33 creates an ID list that describes cell information of each of the cells CA, CB, and CC with IDs = D001 to D004 and the aggregate cell CX with ID = D005. Although not illustrated, an ID list for the cells CA, CB, and CC with IDs = A001, B001, and C001 is also created from the first plate group. Furthermore, the cooperation processing unit 33 creates a list of cooperation information that associates IDs = A001, B001, and C001 as the first identification codes with IDs = D001 to D004 and D005 as the second identification codes, respectively. This cooperation information list is inter-plate transfer information indicating a transfer path of the cells CA, CB, and CC from the first plate group to the second plate 5. Further, the cooperation information of D005 is synthesis information indicating formation of the aggregate cell CX by a plurality of the cells CA, CB, and CC.

When a grid image in which the aggregate cell CX is recognized on Day 5 of the second plate 5 is set as a focus target and a command to display a parent cell constituting the aggregate cell CX is given, the display control unit 21 causes the monitor 16 to display a tree diagram illustrated in Fig. 10. The tree diagram of Fig. 10 is a lineage-style tree diagram illustrating that the aggregate cell CX is formed by the cells CA, CB, CC picked from the first plate group and transferred to the second plate 5.

Such a tree diagram can be displayed on the monitor 16 since an ID list is created by assignment of an identification code to each cell before and after generation of an aggregate cell, and a cooperation information list that associates IDs =A001, B001, and C001 of the first plate 4 with ID=D005 of the second plate is created. The user who visually recognizes the monitor 16 can grasp a generation process, like information of the constituent cells CA, CB, and CC, with respect to the aggregate cell CX as a focus target.

The embodiment described above includes an invention shown below.

A cell handling apparatus according to one aspect of the present invention includes: a first plate including a first cell containing portion capable of containing a cell; a second plate including a second cell containing portion capable of containing a cell; a head that sucks a cell from the first cell containing portion, discharges a sucked cell to the second cell containing portion, and transfers a cell between the first plate and the second plate; and a recognition processing unit configured to recognizes a cell in the first cell containing portion and the second cell containing portion, in which the recognition processing unit performs processing of assigning a first identification code for recognizing a first cell contained in the first cell containing portion, a second identification code for recognizing the first cell transferred to the second cell containing portion, and cooperation information for associating the first identification code and the second identification code.

According to this aspect, an identification code is given for each plate with respect to the first cell transferred from the first plate to the second plate. That is, the first identification code is assigned when the first cell is contained in the first cell containing portion, and the second identification code is assigned when the first cell is contained in the second cell containing portion. Then, cooperation information for associating the first identification code and the second identification code is assigned. Therefore, since a lineage can be traced even in a case where a cell is transferred between plates, the cell can be accurately managed.

In the above cell handling apparatus, in a case where the first cell is divided into at least a second cell and a third cell in the second cell containing portion, the recognition processing unit desirably assigns the cooperation information and assigns the second identification codes different from each other to the second cell and the third cell.

According to this aspect, in a case where a cell divides after transfer to the second plate, to each of the divided cells, an identification code is assigned, and cooperation information is also assigned. Therefore, even if cell division occurs, it is possible to trace which cell of the first plate each divided cell originates from.

The above cell handling apparatus desirably further includes: a display unit configured to display information regarding a cell to be handled; and a display control unit configured to control a display operation on the display unit, and the display control unit desirably causes the display unit to display, in a form of a tree diagram, inter-plate transfer information including transfer of the first cell from the first plate to the second plate and cell division information including division into the second cell and the third cell based on information assigned by the recognition processing unit.

According to this aspect, the user who visually recognizes the display unit can grasp at a glance a differentiation process of a cell transferred from the first plate to the second plate.

The above cell handling apparatus desirably further includes: a display unit configured to display information regarding a cell to be handled; and a display control unit configured to control a display operation on the display unit, and the display control unit desirably causes the display unit to display inter-plate transfer information including transfer of a designated cell between the first plate and the second plate and information linearly showing a transition between the designated cell and the first cell based on information assigned by the recognition processing unit.

According to this aspect, the user who visually recognizes the display unit can linearly grasp a parent-child relationship of one cell that divides in the second plate by tracing back to the first plate.

In the above cell handling apparatus, in a case where a plurality of the first plates exist, a plurality of the first cells are transferred from each of the first cell containing portions to one of the second cell containing portions of the second plate, and one aggregate cell is generated by a plurality of the first cells, the recognition processing unit desirably assigns the cooperation information and assigns the second identification code to the aggregate cell.

According to this aspect, in a case where one aggregate cell is generated by a plurality of first cells, the aggregate cell and a plurality of the first cells are associated with each other by the cooperation information. Therefore, it is possible to manage which first cell of a plurality of the first plates an aggregate cell of the second plate is composed of.

The above cell handling apparatus desirably further includes: a display unit configured to display information regarding a cell to be handled; and a display control unit configured to control a display operation on the display unit, and the display control unit desirably causes the display unit to display, in a form of a tree diagram, inter-plate transfer information including transfer of a plurality of the first cells between the first plate and the second plate and synthesis information indicating formation of the aggregate cell by a plurality of the first cells based on information assigned by the recognition processing unit.

According to this aspect, the user who visually recognizes the display unit can grasp a cell constituting an aggregate cell generated in the second plate by tracing back to the first plate.

According to the present invention, in the cell handling apparatus that transfers a cell between a plurality of plates, management of a cell can be performed accurately.

This application is based on Japanese Patent application No. 2025-12180 filed in Japan Patent Office on January 28, 2025, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. A cell handling apparatus (1) comprising:
a first plate (41) including a first cell containing portion (41) capable of containing a cell (C);
a second plate (5) including a second cell containing portion (51) capable of containing a cell (C);
a head (14) that sucks a cell (C) from the first cell containing portion (41), discharges a sucked cell to the second cell containing portion (51), and transfers a cell (C) between the first plate (4) and the second plate (5); and
a recognition processing unit (3) configured to recognize a cell (C) in the first cell containing portion (41) and the second cell containing portion (51), wherein
the recognition processing unit (3) performs processing of assigning a first identification code (A001) for recognizing a first cell contained in the first cell containing portion (41), a second identification code (B001~B004) for recognizing the first cell transferred to the second cell containing portion (51), and cooperation information for associating the first identification code (A001) and the second identification code (B001~B004).

2. The cell handling apparatus (1) according to claim 1, wherein
in a case where the first cell is divided into at least a second cell and a third cell in the second cell containing portion (51),
the recognition processing unit (3) assigns the cooperation information and assigns the second identification codes (B001~B004) different from each other to the second cell and the third cell.

3. The cell handling apparatus (1) according to claim 2, further comprising:
a display unit (16) configured to display information regarding a cell (C) to be handled; and
a display control unit (21) configured to control a display operation on the display unit (16), wherein
the display control unit (21) causes the display unit (16) to display, in a form of a tree diagram, inter-plate transfer information including transfer of the first cell from the first plate (4) to the second plate (5) and cell division information including division into the second cell and the third cell based on information assigned by the recognition processing unit (3).

4. The cell handling apparatus (1) according to claim 2, further comprising:
a display unit (16) configured to display information regarding a cell (C) to be handled; and
a display control unit (21) configured to control a display operation on the display unit (16), wherein
the display control unit (21) causes the display unit (16) to display inter-plate transfer information (FIG.9) including transfer of a designated cell between the first plate (4) and the second plate (5) and information linearly showing a transition between the designated cell and the first cell based on information assigned by the recognition processing unit (3).

5. The cell handling apparatus (1) according to claim 1, wherein
in a case where a plurality of the first plates (4A,4B,4C) exist, a plurality of the first cells are transferred from each of the first cell containing portions (41A,41B,41C) to one of the second cell containing portions (51) of the second plate (5), and one aggregate cell (CX) is generated by a plurality of the first cells (CA,CB,CC),
the recognition processing unit (3) assigns the cooperation information and assigns the second identification code (D005) to the aggregate cell (CX).

6. The cell handling apparatus according to claim 5, further comprising:
a display unit (16) configured to display information regarding a cell (C) to be handled; and
a display control unit (21) configured to control a display operation on the display unit (16), wherein
the display control unit (21) causes the display unit (16) to display, in a form of a tree diagram, inter-plate transfer information (FIG.10) including transfer of a plurality of the first cells (CA,CB,CC) between the first plate (4) and the second plate (5) and synthesis information indicating formation of the aggregate cell (CX) by a plurality of the first cells (CA,CB,CC) based on information assigned by the recognition processing unit (3).
